# EUROPEAN PATENT SPECIFICATION

(11) **EP 2 421 387 B1**
(45) Date of publication and mention of the grant of the patent: **23.11.2016**
(21) Application number: 10719668.5
(22) Date of filing: 23.04.2010
(51) Int. Cl.: A23L 29/231, A23L 29/20, A23L 19/00, A23C 13/16, A23C 9/133, A23C 9/137, A23C 17/00

(54) **SHELF-STABLE FERMENTED DAIRY PRODUCTS AND METHODS OF MAKING SAME**
LAGERSTABILE FERMENTIERTE MILCHPRODUKTE UND VERFAHREN ZU IHRER HERSTELLUNG
PRODUITS LAITIERS FERMENTÉS DE LONGUE CONSERVATION ET LEURS PROCÉDÉS DE FABRICATION

(30) Priority: 24.04.2009 US 172443 P
(43) Date of publication of application: 29.02.2012
(73) Proprietor: Nestec S.A., 1800 Vevey (CH)
(72) Inventor: WIESSEL, Ana Lucia, Spring Lake Michigan 49456 (US); ZERLAUT, Allen Bruce, Fremont Michigan 49412-9111 (US); WELCH, Frank Karl, Kentwood Michigan 49512 (US)
(74) Representative: Kingsbury, Oliver William
(86) International application number: PCT/US2010/032263
(87) International publication number: WO 2010/124224

(56) References cited:
- WO-A1-2008/113663
- FR-A1- 2 887 121
- FR-A1- 2 892 270
- US-A- 4 748 026
- US-A- 4 971 810
- US-A1- 2006 057 247

## Description

### BACKGROUND

The present disclosure generally relates to health and nutrition. More specifically, the present disclosure relates to methods of making shelf-stable fermented dairy products, and to shelf-stable fermented dairy products prepared by these methods.

There are many refrigerated food products currently on the market. Refrigeration is the process of cooling or freezing the food product to lower temperatures so as to extend the life of the food product. During storage, bacteria within food products can cause the food product to spoil over time. By refrigerating, a food product can be maintained without spoiling for extended periods of time such as weeks or months. Typical food products requiring refrigeration include meat and dairy products including fermented dairy products such as yogurt. However, food products that require refrigeration are generally more costly to store than non-refrigerated foods due to the energy costs associated with refrigeration or freezing.

Shelf-stable foods are foods that would normally be stored refrigerated but have been processed so that they can be safely stored at room or ambient temperature for long shelf life. Various food preservation and packaging techniques are used to extend a food's shelf life. Some of these techniques include decreasing the amount of available water in a food product, increasing its acidity, or irradiating or otherwise sterilizing the food product and then sealing it in an air-tight container. For some foods alternative ingredients can be used. However, different types of food products each required specific techniques to increase the food's shelf life without unacceptably changing its taste or texture.

A fermented dairy product such as yogurt is very susceptible to protein coagulation when heated following the fermentation process. Furthermore, a fermented dairy product introduces a multitude of challenges in maintaining shelf-stability while providing the appropriate taste and texture profiles. Therefore, there is a need for a shelf-stable fermented dairy product that is appealing to a consumer and does not need to be refrigerated.

Documents WO 2008/113663 A1, US 2006/057247 A1, US 4 971 810 A, FR 2 887 121 A1 and FR 2 892 270 A1 disclose dairy products comprising a fruit component and a stabiliser. Document US 4 748 026 A1 discloses a process for the production of a shelf stable yoghurt.

### SUMMARY

Shelf-stable fermented dairy products and methods of making the shelf-stable fermented dairy products are provided. In a general embodiment, the present disclosure provides a method of making a shelf-stable fermented dairy product which comprises a method of making a shelf-stable fermented dairy product, the method comprising:
a. adding a stabilizer comprising a high gelling whey protein concentrate or a hydrocolloid selected from the group consisting of pectin, gelatin, carrageenan, agar, acacia gum, sodium alginate, xanthan gum, locust bean gum and carboxymethyl cellulose or a mixture thereof, to a fermented dairy component under shear at a blending rate of from 10 to 1000 rpm at a temperature of from 0.56 to 18.3 °C (33 to 65 °F) to create a shelf-stable fermented dairy mixture;
b. homogenizing the fermented dairy mixture at a temperature of from 0.56 to 73.9 °C (33 to 165 °F) in a single or dual stage homogenizer with a pressure range from 3.45 to 27.58 MPa (500 to 4000 psi);
c. adding a puree composition to the fermented dairy mixture at a blending rate of from 10 to 1000 rpm at a temperature of from 0.56 to 73.9 °C (33 to 165 °F); and
d. heat processing the fermented dairy mixture to a temperature in the range of 85 to 116 °C (185 to 240 °F) for 10 seconds to 40 minutes, to render the fermented dairy mixture commercially sterile to form the shelf-stable fermented dairy product.

The invention also provides a shelf-stable fermented dairy product prepared according to the method of any of claims 1 to 15.

In an embodiment of the method, the shelf-stable fermented dairy product has a flavor liking score of at least 5 based on a 9-point hedonic scale of a quantitative central location test. The shelf-stable fermented dairy product can have a sweetness liking score of at least 5 based on a 9-point hedonic scale of a quantitative central location test. The shelf-stable fermented dairy product can have a tartness liking score of at least 5 based on a 9-point hedonic scale of a quantitative central location test. In addition, the shelf-stable fermented dairy product can have a texture liking score of at least 5 based on a 9-point hedonic scale of a quantitative central location test.

In an embodiment of the method, adding the stabilizer to the fermented dairy component under shear comprises stabilizing proteins in the fermented dairy
component by coating with the stabilizer. The fermented dairy mixture can be heated to a temperature above 93.3°C (200 °F). In addition, the method can be performed under aseptic conditions.

An advantage of the present disclosure is to provide an improved shelf-stable fermented dairy product that is shelf-stable for at least 3 months or longer.
Yet another advantage of the present disclosure is to provide an improved method of making a shelf-stable fermented dairy product.

Still another advantage of the present disclosure is to provide a commercially sterile product that is not grainy and maintains this characteristic over the shelf life of the product.

Another advantage of the present disclosure is to provide a method for making shelf-stable fermented dairy products that is easily adaptable to commercial processes typically in place for heat processed dairy-based products (e.g., such a pudding).

Yet another advantage of the present disclosure is to provide a method for making shelf-stable fermented dairy products having the ability to add a variety of other ingredients to the shelf-stable fermented dairy product without impacting the finished product stability as it relates to the protein matrix of the shelf-stable fermented dairy product.

Additional features and advantages are described herein, and will be apparent from the following Detailed Description.

### DETAILED DESCRIPTION

Shelf-stable fermented dairy products and methods of making the shelf-stable fermented dairy products are provided.

In one embodiment, the present disclosure provides a method of making a shelf-stable fermented dairy product. The method comprises adding a physical or chemical stabilizer to a fermented dairy component under shear to create a shelf-stable fermented dairy mixture under a temperature range from 0.56°C-18.3°C (33-65F) at a blending range from 10 to 1000 rpm, preferably from about 50 to 500rpm and most preferably from about 100 to about 300 rpm., homogenizing the fermented dairy mixture under a temperature range of from about 0.56°C (33F) to about 73.9°C (165F), preferably about 0.56°C (33F) to about 37.8°C (100F) and most preferably from about 0.56° (33F) to about 15.6°C (60F) and in a single or dual stage homogenizer with pressure range from about 3.45MPa (500 psi) to about 27.58MPa (4000 psi), preferably from about 3.45MPa (500 psi) to about 20.68MPa (3000 psi), and most preferably from about 3.45MPa (500 psi) to about 10.34MPa (1500 psi), adding a puree composition to the fermented dairy mixture under a temperature range from about 0.56°C (33F) to about 73.9°C (165 F) at blending range from 10 to 1000rpm, and heat processing the shelf-stable fermented dairy mixture to render the shelf-stable fermented dairy mixture commercially sterile to form the shelf-stable fermented dairy product in a range of from about 10 seconds to about 40 minutes, at the temperature range of about 85°C (185F) to about 116°C (240F). The method can be performed under aseptic conditions.

The present method unexpectedly creates an improved shelf stable dairy product with improved taste, viscosity and texture. Specifically, refrigerated dairy products coagulate over time and temperature and need to be controlled to obtain the correct viscosity for the end product. High sheer and heat are not necessary and not preferred in the prior art methods since natural proteins create viscosity and thickness which coagulate and form a matrix to build the texture and viscosity of the final product. The method of the present invention surprisingly provided improved viscosity, texture and taste. While viscosity alone may be adjustable in the prior art refrigerated methods, the combination of the viscosity and texture of the present invention provides a surprisingly improved and preferred composition.

The first part of the method involves "stabilizing" protein in the shelf-stable fermented dairy component by coating it with a suitable hydrocolloid (e.g., pectin) or a high gelling whey protein concentrate followed by homogenization of the shelf-stable fermented dairy mixture. This allows the shelf-stable fermented dairy mixture to be heated to sterilization temperatures (e.g., above 85°C (185 °F)) without coagulating the protein thereby resulting in a smooth textured fermented dairy product.

In an embodiment of the method, one or more thickeners can include but are not limited to physically or chemically modified flours and/or starches from sources such as rice, wheat, oat, barley, tapioca, quinoa, rye, amaranth, corn, or potatoe. Flavors and/or colors are added to the fermented dairy mixture before the heat processing. The shelf-stable fermented dairy component can be yogurt, sour cream, buttermilk or a combination thereof.

Embodiments of the present disclosure advantageously provide the capability to produce a commercially sterile, shelf-stable fermented dairy product that is not grainy while maintaining this characteristic over the shelf life of the product. Available commercial processes typically in place for heat processed, dairy-based products (e.g., such a pudding) can be used to make the shelf-stable fermented dairy products. Various ingredients can be added to the shelf-stable fermented dairy products during the manufacturing process without impacting finished product stability as it relates to the protein matrix of the shelf-stable fermented dairy products.

The shelf-stable fermented dairy products can be shelf-stable with developmentally appropriate textures and taste profiles. The shelf-stable fermented dairy product includes a fermented dairy component, a physical or chemical stabilizer, and a puree composition. The fermented dairy component can be, for example, dehydrated or fresh yogurt, sour cream, buttermilk, kefir, cheese, or a combination thereof. Other suitable shelf-stable fermented dairy components can also be used to make the shelf-stable fermented dairy products in embodiments of the present disclosure.

As used herein, the term "shelf-stable" means capable of being stored at room temperature (e.g., about 20 °C to about 25 °C) for long periods (e.g., more than 3 months) without becoming spoiled or rotten. Typical fermented dairy products normally need to be stored refrigerated, but the shelf-stable fermented dairy products in embodiments of the present disclosure have been processed so that they can be safely stored in a sealed container at room or ambient temperature for a usefully along shelf life without unacceptable changing their taste or texture. The fermented dairy product produced can be shelf-stable, for example, for more than 3 months, 6 months, 12 months, 18 mouths, etc.

In an embodiment, the shelf-stable fermented dairy product of the present invention has a taste and flavor profile that yields a liking score from a sensory perspective that is significantly higher than other shelf stable dairy compositions and refrigerated dairy compositions (e.g., obtains or receives from a consumer) a flavor linking score of at least 5, 6, 7, 8 or 9 based on a 9-point hedonic scale of a quantitative central location test. The 9-point hedonic scale is one of the most widely used stale for measuring food acceptability. For example, the 9-point hedonic scale assigns points 1-9 based on user preferences for a food product as follows: Like Extremely - 9; Like Very Much - 8; Like Moderately - 7; Like Slightly - 6; Neither Like nor Dislike - 5; Mislike Slightly - 4; Dislike Moderately - 3; Dislike Very Much - 2; and Dislike Extremely - 1.

Central location tests are product marketing tests performed in controlled environments, contrary to home-user tests, which take place where the products would actually be used. Central location tests can be conducted in a premises such as a room in a shopping mall. Consumers can be recruited to participate in a research product on the shopping mall and the research can be conducted and completed at that time. The consumers can be children or adults. The number of consumers can vary depending on the statistical analysis performed. It should be appreciated that the number of consumers should be enough to provide a statistically relevant test.

The shelf-stable fermented dairy product can have a score of at least 5, 6, 7, 8 or 9 for other characteristics based on a 9-point hedonic scale of quantitative central location test. For example, the characteristics can include appearance liking, color linking, flavor liking, fruit flavor liking, sweetness liking, tartness liking, texture liking or consistency liking.

In an embodiment, the stabilizer is a physical or chemical stabilizer and is a hydrocolloid or a high gelling whey protein concentrate. The hydrocolloid can be pectin, gelatin, carrageenan, agar, acacia gum, sodium alginate, xanthan gum, locust bean gum, carboxymethyl cellulose (CMC) or a combination thereof. The stabilizer can range from about 0.001% to about 10% by weight, preferably from about 0.01% to 5% and most preferably from about 0.2% to about 0.5%.

In an embodiment, the shelf-stable fermented dairy product has a pH ranging from about 3.8 to about 4.3, preferably from about 3.9 to 4.3 and most preferably about 4.1 to 4.3.

The present invention offers a surprisingly significant difference and preference in viscosity and texture as seen in Tables 1- below. Viscosity is measured using a Brookfield RV #6 Spindle at 5RPM, 10 seconds and ranges from about at least 20000 mPa.s (centipoise), preferably from about 30000 mPa.s (centipoise) to about 70000 mPa.s (centipoise) and most preferably about 35000 mPa.s (centipoise) to about 60000 mPa.s (centipoise). Texture is measured using a TMS-Pro Texture Analzyer-Serial #07-1066-08 and ranges from about 3.000 Newtons to about 5.000, preferably from about 3.200 to about 4.800 and most preferably from about 3.400 to about 4.500.

In a comparative analysis of flavored yogurts of the present invention (A) with yogurts of similar flavor in another shelf stable yogurt product (B) and a refrigerated yogurt product (C), the results showed a statistically significant difference between the viscosity and texture of the present invention and the two other products, as detailed in Tables 1-4 below.

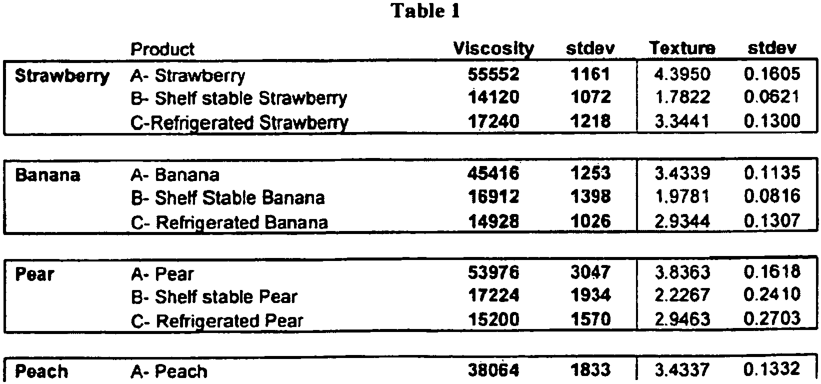

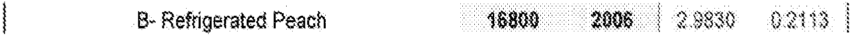

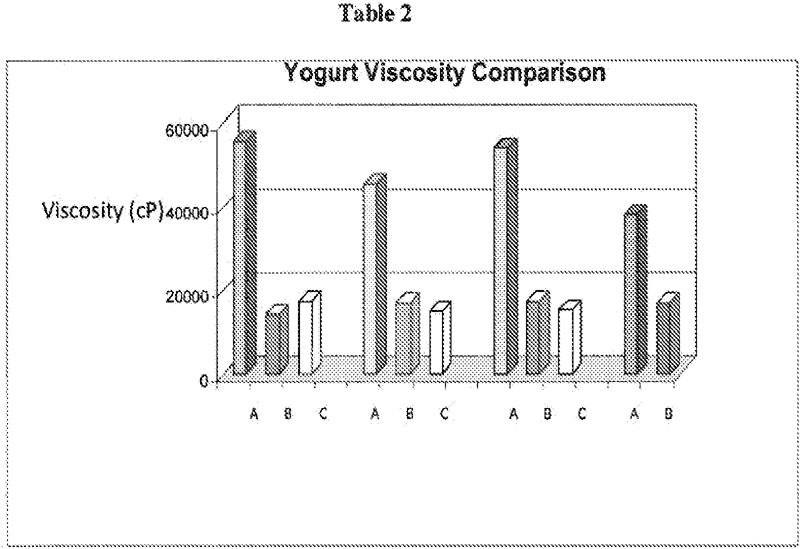

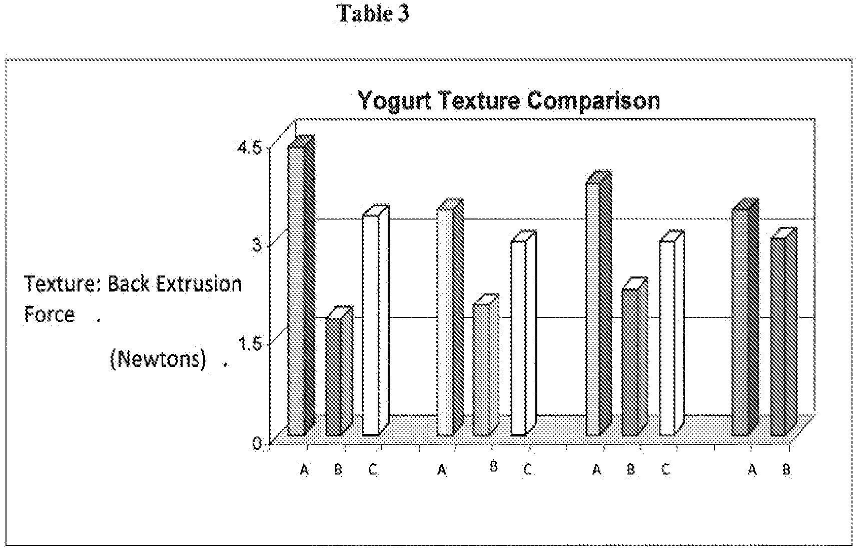

**Table 4**

| Texture-Strawberry | | | |
|---|---|---|---|
| | Brand | | |
| | | | |
| | A | B | C |
| Texture | 4.40 BC | 1.78 | 3.3 B |

| Texture-Banana | | | |
|---|---|---|---|
| | Brand | | |
| | | | |
| | A | B | C |
| Texture | 3.43 BC | 1,98 | 2.93 B |

| Texture-Pear | | | |
|---|---|---|---|
| | | | |
| | | | |
| | A | B | C |
| Texture | 3.84 BC | 2.23 | 2.95 B |

| Texture-Peach | | | |
|---|---|---|---|
| | Brand | | |
| | | | |
| | A | B | |
| Texture | 3.43 B | 2.98 | |

| Viscosity-Strawberry | | | |
|---|---|---|---|
| | Brand | | |
| | | | |
| | A | B | C |
| | Viscosity 55552 BC | 14120 | 17240 B |

| Viscosity-Banana | | | |
|---|---|---|---|
| | Brand | | |
| | | | |
| | A | B | C |
| Viscosity | 45416 BC | 16912 C | 14928 |

| Viscosity-Pear | | | |
|---|---|---|---|
| | Brand | | |
| | | | |
| | A | B | C |
| Viscosity | 53976 BC | 17224 C | 15200 |

| Viscosity-Peach | | | |
|---|---|---|---|
| | Brand | | |
| | | | |
| | A | B | |
| Viscosity | 38064 B | 16800 | |

In the present invention, sensory tests were conducted by trained sensory panelists with a Descriptive Analysis using a 100 point Unstructured Line Scale.

The shelf-stable fermented dairy product can include also include acidulants including but limited to lactic acid, malic acid, citric acid, tartaric acid, phosphoric acid, glocono delta lactone in an amount of about 0.01% to about 2% by weight, preferably from about 0.1-1% by weight.

In an embodiment, the composition can include sugar in an amount up to about 20% by weight, preferably from about 3% to 15% by weight, and most preferably from about 5% to about 10% by weight. The shelf-stable fermented dairy product can also be sugar free and include sugarless sweeteners such as maltitol, mannitol, xylitol, hydrogenated starch hydrolysates, sorbitol, lactitol, erythritol and the like, alone or in combination.

High intensity artificial or natural sweeteners can also be used in the shelf-stable fermented dairy product. Preferred sweeteners include, but are not limited to sucralose, aspartame, salts of acesulfame, alitame, saccharin and its salts, cyclamic acid and its salts, glycyrrhizin, stevioside, dihydrochalcones, thaumatin, monellin, and the like, alone or in combination.

In an embodiment, the puree composition includes a pureed fruit including but not limited to apple, orange, pear, peach, strawberry, banana, cherry, pineapple, kiwi, grape, blueberry, raspberry, mango, guava, cranberry, blackberry or a combination thereof. The fruit can be present in an amount ranging from about 0% to about 80% by weight, preferably from about 3% to about 20% by weight and most preferably from about 5% to about 10% by weight. Flavor components in general can range from about 0% to about 10%, preferably from about 0.001% to about 5% and most preferably from about 0.1% to about 4% by weight.

In an embodiment, the composition can include a vegetable ingredient selected from the group including but not limited to sweet potatoes, carrots, peas, green beans and squash.

In an embodiment, the shelf-stable fermented dairy product further includes one or more prebiotics. As used herein, a prebiotic is a selectively fermented ingredient that allows specific changes, both in the composition and/or activity in the gastrointestinal microflora, that confers benefits upon host well-being and health. Non-limiting examples of prebiotics include fructooligosaccharides, inulin, lactulose, galactooligosaccharides, acacia gum, soyoligosaccharides, xylooligosaccharides, isomaltooligosaccharides, gentiooligosaccharides, lactosucrose, glucooligosaccharides, pecticoligosaccharides, resistant starches, sugar alcohols or a combination thereof.

In an embodiment, the shelf-stable fermented dairy product further includes one or more probiotics. As used herein, probiotics are defined as microorganisms (e.g., live) that could confer health benefits on the host when administered in adequate amounts. Non-limiting examples of probiotics include Saccharomyces, Debaromyces, Candida, Pichia, Torulopsis, Aspergillus, Rhizopus, Mucor, Penicillium, Torulopsis, Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus, Lactobacillus or a combination thereof.

In another embodiment, the shelf-stable fermented dairy product further includes one or more amino acids. Non-limiting examples of amino acids include Isoleucine, Alanine, Leucine, Asparagine, Lysine, Aspartate, Methionine, Cysteine, Phenylalanine, Glutamate, Threonine, Glutamine, Tryptophan, Glycine, Valine, Proline, Serine, Tyrosine, Arginine, Histidine or a combination thereof.

In an embodiment, the shelf-stable fermented dairy product further includes one or more symbiotics, phytonutrients, antioxidants, vitamins and/or minerals. As used herein, a symbiotic is a supplement that contains both a prebiotic and a probiotic that work together to improve the microflora of the intestine. Non-limiting examples of phytonutrients include quercetin, curcumin and limonin. Antioxidants are molecules capable of slowing or preventing the oxidation of other molecules. Non-limiting examples of antioxidants include vitamin A, carotenoids, vitamin C, vitamin E, selenium, flavonoids, polyphenols, lycopene, lutein, lignan, coenzyme Q10 ("CoQ10") and glutathione.

Non-limiting examples of vitamins may include Vitamins A, B-complex (such as B-1, B-2, B-6 and B-12), C, D, E and K, niacin and acid vitamins such as pantothenic acid and folic acid and biotin. Non-limiting examples of minerals may include calcium, iron, zinc, magnesium, iodine, copper, phosphorus, manganese, potassium, chromium, molybdenum, selenium, nickel, tin, silicon, vanadium and boron.

### EXAMPLES

By way of example and not limitation, the following examples are illustrative of various embodiments of the present disclosure. The formulations below are provided for exemplification only, and they can be modified by the skilled artisan to the necessary extent, depending on the special features that are looked for.

### EXAMPLE 1

**Yogurt Blends - Banana**

| **Material Name** | **Percent** |
|---|---|
| Full Fat Yogurt, Refrigerated | 85.06 |
| Sugar | 5.54 |
| Banana Puree, Deseeded | 5.00 |
| Tapioca Starch Physically Treated | 3.50 |
| Flavor, Banana | 0.54 |
| Pectin | 0.35 |
| Color Turmeric | 0.003 |
| Citric Acid | 0.01 |

**Yogurt Blends -Peach**

| **Material Name** | **Percent** |
|---|---|
| Full Fat Yogurt, Refrigerated | 85.15 |
| Sugar | 5.55 |
| Peach Puree Concentrate | 3.04 |
| Water to reconstitute puree | 1.86 |
| Tapioca Starch Physically Treated | 3.50 |
| Flavor, Peach | 0.54 |
| Pectin | 0.35 |
| Color, Annatto | 0.01 |
| Citric Acid | 0.01 |

It should be understood that various changes and modifications to the presently preferred embodiments described herein will be apparent to those skilled in the art. Such changes and modifications can be made without departing from the scope of the present subject matter and without diminishing its intended advantages. It is therefore intended that such changes and modifications be covered by the appended claims.

## Claims

1. A method of making a shelf-stable fermented dairy product, the method comprising:
a. adding a stabilizer comprising a high gelling whey protein concentrate or a hydrocolloid selected from the group consisting of pectin, gelatin, carrageenan, agar, acacia gum, sodium alginate, xanthan gum, locust bean gum and carboxymethyl cellulose or a mixture thereof, to a fermented dairy component under shear at a blending rate of from 10 to 1000 rpm at a temperature of from 0.56 to 18.3 °C (33 to 65 °F) to create a shelf-stable fermented dairy mixture;
b. homogenizing the fermented dairy mixture at a temperature of from 0.56 to 73.9 °C (33 to 165 °F) in a single or dual stage homogenizer with a pressure range from 3.45 to 27.58 MPa (500 to 4000 psi);
c. adding a puree composition to the fermented dairy mixture at a blending rate of from 10 to 1000 rpm at a temperature of from 0.56 to 73.9 °C (33 to 165 °F); and
d. heat processing the fermented dairy mixture to a temperature in the range of 85 to 116 °C (185 to 240 °F) for 10 seconds to 40 minutes, to render the fermented dairy mixture commercially sterile to form the shelf-stable fermented dairy product.

2. The method of Claim 1, wherein adding the stabilizer to the fermented dairy component under shear comprises stabilizing proteins in the fermented dairy component by coating with the physical stabilizer.

3. The method of Claim 1, wherein the blending rate in step (a) is from 50 to 500 rpm, or from 100 to 300 rpm.

4. The method of Claim 1, wherein the method is performed under aseptic conditions.

5. The method of Claim 1, wherein at least one of thickeners, flavors, sweeteners, acidulants and colors is added to the mixture before the heat processing.

6. The method of claim 5, wherein the thickener comprises a physically or chemically modified flour or starch from rice, wheat, oat, barley, tapioca, quinoa, rye, amaranth, corn, or potato.

7. The method of Claim 1, wherein the shelf-stable fermented dairy product has;
a flavor liking score of at least 5 based on a 9-point hedonic scale of a quantitative central location test;
a sweetness liking score of at least 5 based on a 9-point hedonic scale of a quantitative central location test;
a tartness liking score of at least 5 based on a 9-point hedonic scale of a quantitative central location test; or
a texture liking score of at least 5 based on a 9-point hedonic scale of a quantitative central location test.

8. The method of Claim 1, wherein the shelf-stable fermented dairy component is selected from the group consisting of yogurt, sour cream, buttermilk, kefir, cheese and combinations thereof.

9. The method of Claim 1, wherein the stabilizer comprises pectin.

10. The method of Claim 1, wherein the stabilizer ranges from 0.001% to 10% by weight.

11. The method of Claim 1, wherein the shelf-stable fermented dairy product comprises a pH ranging from 3.8 to 4.3.

12. The method of Claim 1, wherein the puree composition comprises a pureed fruit selected from the group consisting of apple, orange, pear, peach, strawberry, banana, cherry, pineapple, kiwi, grape, blueberry, raspberry, mango, guava, cranberry, blackberry and combinations thereof.

13. The method of Claim 1, wherein the shelf-stable fermented dairy product further comprises a prebiotic, which is optionally selected from the group consisting of partially hydrolyzed guar gum, fructooligosaccharides, inulin, lactulose, galactooligosaccharides, acacia gum, soyoligosaccharides, xylooligosaccharides, isomaltooligosaccharides, gentiooligosaccharides, lactosucrose, glucooligosaccharides, pecticoligosaccharides, resistant starches, sugar alcohols and combinations thereof.

14. The method of Claim 1, wherein the shelf-stable fermented dairy product further comprises a probiotic, which is optionally selected from the group consisting of Saccharomyces, Debaromyces, Candida, Pichia, Torulopsis, Aspergillus, Rhizopus, Mucor, Penicillium, Torulopsis, Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus, Lactobacillus and combinations thereof.

15. The method of Claim 1, wherein the shelf-stable fermented dairy product further comprises;
a component selected from the group consisting of symbiotics, phytonutrients and combinations thereof;
an amino acid optionally selected from the group consisting of Isoleucine, Alanine, Leucine, Asparagine, Lysine, Aspartate, Methionine, Cysteine, Phenylalanine, Glutamate, Threonine, Glutamine, Tryptophan, Glycine, Valine, Proline, Serine, Tyrosine, Arginine, Histidine and combinations thereof;
an antioxidant;
a vitamin; or
a mineral.

16. A shelf-stable fermented dairy product prepared according to the method of any of claims 1 to 15.

17. The shelf-stable fermented dairy product of claim 16, having a viscosity of at least 20,000 mPa.s (centipoise) using a Brookfield RV #6 Spindle at 5 RPM for 10 seconds.

## Patentansprüche

1. Verfahren zum Herstellen eines bei Raumtemperatur haltbaren fermentierten Milchprodukts, wobei das Verfahren umfasst:
a. Zugeben eines Stabilisators, der ein stark gelierendes Molkenproteinkonzentrat oder ein Hydrokolloid umfasst, das aus der Gruppe bestehend aus Pektin, Gelatine, Carrageen, Agar, Gummiarabikum, Natriumalginat, Xanthan, Johannisbrotkernmehl und Carboxymethylcellulose oder einem Gemisch daraus ausgewählt ist, zu einer fermentierten Milchkomponente unter Scherung mit einer Mischgeschwindigkeit von 10 bis 1000 U/min bei einer Temperatur von 0,56 bis 18,3 °C (33 bis 65 °F), um ein bei Raumtemperatur haltbares fermentiertes Milchgemisch zu erzeugen;
b. Homogenisieren des fermentierten Milchgemischs bei einer Temperatur von 0,56 bis 73,9 °C (33 bis 165 °F) in einem einstufigen oder zweistufigen Homogenisator mit einem Druckbereich von 3,45 bis 27,58 MPa (500 bis 4000 psi);
c. Zugeben einer Püreezusammensetzung zu dem fermentierten Milchgemisch bei einer Mischgeschwindigkeit von 10 bis 1000 U/min bei einer Temperatur von 0,56 bis 73,9 °C (33 bis 165 °F); und
d. Wärmebehandlung des fermentierten Milchgemischs auf eine Temperatur im Bereich von 85 bis 116 °C (185 bis 240 °F) für 10 Sekunden bis 40 Minuten, um das fermentierte Milchgemisch technisch steril zu machen, um das bei Raumtemperatur haltbare fermentierte Milchprodukt zu bilden.

2. Verfahren nach Anspruch 1, wobei das Zugeben des Stabilisators zu der fermentierten Milchkomponente unter Scherung das Stabilisieren von Proteinen in der fermentierten Milchkomponente durch Beschichten mit dem physischen Stabilisator umfasst.

3. Verfahren nach Anspruch 1, wobei die Mischgeschwindigkeit in Schritt (a) zwischen 50 bis 500 U/min, oder zwischen 100 bis 300 U/min liegt.

4. Verfahren nach Anspruch 1, wobei das Verfahren unter aseptischen Bedingungen durchgeführt wird.

5. Verfahren nach Anspruch 1, wobei mindestens eines von Verdickungsmitteln, Aromastoffen, Süßstoffen, Säuerungsmitteln und Farbstoffen vor der Wärmebehandlung zu dem Gemisch zugegeben wird.

6. Verfahren nach Anspruch 5, wobei das Verdickungsmittel ein physikalisch oder chemisch modifiziertes Mehl oder eine Stärke von Reis, Weizen, Hafer, Gerste, Tapioka, Quinoa, Roggen, Amaranth, Mais oder Kartoffeln umfasst.

7. Verfahren nach Anspruch 1, wobei das bei Raumtemperatur haltbare fermentierte Milchprodukt folgendes aufweist:
eine Punktzahl bei der Geschmacksbewertung von mindestens 5, basierend auf einer hedonischen 9-Punkte-Skala eines quantitativen Central Location-Tests;
eine Punktzahl bei der Süßebewertung von mindestens 5, basierend auf einer hedonischen 9-Punkte-Skala eines quantitativen Central Location-Tests;
eine Punktzahl bei der Säurebewertung von mindestens 5, basierend auf einer hedonischen 9-Punkte-Skala eines quantitativen Central Location-Tests; oder
eine Punktzahl bei der Texturbewertung von mindestens 5, basierend auf einer hedonischen 9-Punkte-Skala eines quantitativen Central Location-Tests.

8. Verfahren nach Anspruch 1, wobei die bei Raumtemperatur haltbare fermentierte Milchkomponente aus der Gruppe bestehend aus Joghurt, saurer Sahne, Buttermilch, Kefir, Käse und Kombinationen daraus ausgewählt ist.

9. Verfahren nach Anspruch 1, wobei der Stabilisator Pektin umfasst.

10. Verfahren nach Anspruch 1, wobei der Stabilisator zwischen 0,001 bis 10 Gew.-% liegt.

11. Verfahren nach Anspruch 1, wobei das bei Raumtemperatur haltbare fermentierte Milchprodukt einen pH-Wert zwischen 3,8 und 4,3 umfasst.

12. Verfahren nach Anspruch 1, wobei die Püreezusammensetzung eine pürierte Frucht umfasst, die aus der Gruppe bestehend aus Apfel, Orange, Birne, Pfirsich, Erdbeere, Banane, Kirsche, Ananas, Kiwi, Traube, Heidelbeere, Himbeere, Mango, Guave, Cranberry, Brombeere und Kombinationen davon ausgewählt ist.

13. Verfahren nach Anspruch 1, wobei das bei Raumtemperatur haltbare fermentierte Milchprodukt ferner ein Präbiotikum umfasst, das optional aus der Gruppe bestehend aus teilweise hydrolysiertem Guarkernmehl, Fructooligosacchariden, Inulin, Lactulose, Galactooligosacchariden, Gummiarabikum, Sojaoligosacchariden, Xylooligosacchariden, Isomaltooligosacchariden, Gentiooligosacchariden, Lactosucrose, Glucooligosacchariden, Pektin-Oligosacchariden, resistenten Stärken, Zuckeralkoholen und Kombinationen davon ausgewählt ist.

14. Verfahren nach Anspruch 1, wobei das bei Raumtemperatur haltbare fermentierte Milchprodukt ferner ein Probiotikum umfasst, das wahlweise aus der Gruppe bestehend aus Saccharomyces, Debaromyces, Candida, Pichia, Torulopsis, Aspergillus, Rhizopus, Mucor, Penicillium, Bifidobacterium, Bacteroides, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus, Lactobacillus oder Kombinationen davon ausgewählt ist.

15. Verfahren nach Anspruch 1, wobei das bei Raumtemperatur haltbare fermentierte Milchprodukt ferner umfasst:
eine Komponente, die aus der Gruppe bestehend aus Symbiotika, pflanzlichen Inhaltsstoffen und Kombinationen davon ausgewählt sind;
eine Aminosäure, die wahlweise aus der Gruppe bestehend aus Isoleucin, Alanin, Leucin, Asparagin, Lysin, Aspartat, Methionin, Cystein, Phenylalanin, Glutamat, Threonin, Glutamin, Tryptophan, Glycin, Valin, Prolin, Serin, Tyrosin, Arginin, Histidin und Kombinationen davon ausgewählt ist;
ein Antioxidans;
ein Vitamin; oder
einen Mineralstoff.

16. Bei Raumtemperatur haltbares, fermentiertes Milchprodukt, das nach dem Verfahren nach einem der Ansprüche 1 bis 15 hergestellt wurde.

17. Bei Raumtemperatur haltbares fermentiertes Milchprodukt nach Anspruch 16, das eine Viskosität von mindestens 20.000 mPa.s (Centipoise) aufweist, unter Verwendung einer Brookfield RV #6-Spindel bei 5 U/min für 10 Sekunden.

## Revendications

1. Procédé de fabrication d'un produit laitier fermenté à longue durée de conservation, le procédé comprenant :
a. l'ajout d'un agent stabilisant comprenant un concentré de protéines lactosériques à fort pouvoir gélifiant ou un hydrocolloïde choisi dans le groupe constitué de pectine, gélatine, carraghénane, agar, gomme d'acacia, alginate de sodium, gomme de xanthane, gomme de caroube et carboxyméthylcellulose ou un mélange de ceux-ci, à un composant laitier fermenté sous cisaillement à une vitesse de mélange allant de 10 à 1000 tr/min à une température allant de 0,56 à 18,3 °C (33 à 65 °F) de façon à créer un mélange laitier fermenté à longue durée de conservation ;
b. homogénéiser le mélange laitier fermenté à une température allant de 0,56 à 73,9 °C (33 à 165 °F) dans un homogénéiseur à étage unique ou double étage avec une plage de pression de 3,45 à 27,58 MPa (500 à 4000 psi) ;
c. ajouter une composition de purée au mélange laitier fermenté à une vitesse de mélange allant de 10 à 1000 tr/min à une température allant de 0,56 à 73,9 °C (33 à 165 °F) ; et
d. traiter thermiquement le mélange laitier fermenté à une température dans la plage de 85 à 116 °C (185 à 240 °F) pendant 10 secondes à 40 minutes, pour rendre le mélange laitier fermenté commercialement stérile de façon à former le produit laitier fermenté à longue durée de conservation.

2. Procédé selon la revendication 1, dans lequel l'ajout de l'agent stabilisant au composant laitier fermenté sous cisaillement comprend la stabilisation des protéines dans le composant laitier fermenté par revêtement avec l'agent stabilisant physique.

3. Procédé selon la revendication 1, dans lequel la vitesse de mélange dans l'étape (a) va de 50 à 500 tr/min, ou de 100 à 300 tr/min.

4. Procédé selon la revendication 1, où le procédé est effectué dans des conditions aseptiques.

5. Procédé selon la revendication 1, dans lequel au moins l'un parmi des épaississants, des arômes, des édulcorants, des acidulants et des colorants est ajouté au mélange avant le traitement thermique.

6. Procédé selon la revendication 5, dans lequel l'épaississant comprend une farine ou un amidon physiquement ou chimiquement modifié de riz, blé, avoine, orge, tapioca, quinoa, seigle, amarante, maïs, ou pomme de terre.

7. Procédé selon la revendication 1, dans lequel le produit laitier fermenté à longue durée de conservation possède :
une cote d'appréciation de saveur d'au moins 5 sur la base d'une échelle hédoniste à 9 points d'un test quantitatif à localisation centrale ;
une cote d'appréciation de sucrosité d'au moins 5 sur la base d'une échelle hédoniste à 9 points d'un test quantitatif à localisation centrale ;
une cote d'appréciation d'aigreur d'au moins 5 sur la base d'une échelle hédoniste à 9 points d'un test quantitatif à localisation centrale ; ou
une cote d'appréciation de texture d'au moins 5 sur la base d'une échelle hédoniste à 9 points d'un test quantitatif à localisation centrale.

8. Procédé selon la revendication 1, dans lequel le composant laitier fermenté à longue durée de conservation est choisi dans le groupe constitué de yaourt, crème aigre, babeurre, kéfir, fromage et des combinaisons de ceux-ci.

9. Procédé selon la revendication 1, dans lequel l'agent stabilisant comprend de la pectine.

10. Procédé selon la revendication 1, dans lequel l'agent stabilisant va de 0,001 % à 10 % en poids.

11. Procédé selon la revendication 1, dans lequel le produit laitier fermenté à longue durée de conservation comprend un pH allant de 3,8 à 4,3.

12. Procédé selon la revendication 1, dans lequel la composition de purée comprend un fruit en purée choisi dans le groupe constitué de pomme, orange, poire, pêche, fraise, banane, cerise, ananas, kiwi, raisin, myrtille, framboise, mangue, goyave, canneberge, mûre et des combinaisons de ceux-ci.

13. Procédé selon la revendication 1, dans lequel le produit laitier fermenté à longue durée de conservation comprend en outre un prébiotique, qui est facultativement choisi dans le groupe constitué de gomme de guar partiellement hydrolysée, fructo-oligosaccharides, inuline, lactulose, galacto-oligosaccharides, gomme d'acacia, soja-oligosaccharides, xylo-oligosaccharides, isomaltooligosaccharides, gentio-oligosaccharides, lactosaccharose, gluco-oligosaccharides, oligosaccharides pectiques, amidons résistants, alcools de sucre et des combinaisons de ceux-ci.

14. Procédé selon la revendication 1, dans lequel le produit laitier fermenté à longue durée de conservation comprend en outre un probiotique, qui est facultativement choisi dans le groupe constitué de Saccharomyces, Debaromyces, Candida, Pichia, Torulopsis, Aspergillus, Rhizopus, Mucor, Penicillium, Torulopsis, Bifidobacterium, Bactéroïdes, Clostridium, Fusobacterium, Melissococcus, Propionibacterium, Streptococcus, Enterococcus, Lactococcus, Staphylococcus, Peptostrepococcus, Bacillus, Pediococcus, Micrococcus, Leuconostoc, Weissella, Aerococcus, Oenococcus, Lactobacillus et des combinaisons de ceux-ci.

15. Procédé selon la revendication 1, dans lequel le produit laitier fermenté à longue durée de conservation comprend en outre ;
un composant choisi dans le groupe constitué de symbiotiques, phytonutriments et des combinaisons de ceux-ci ;
un acide aminé facultativement choisi dans le groupe constitué d'isoleucine, alanine, leucine, asparagine, lysine, aspartate, méthionine, cystéine, phénylalanine, glutamate, thréonine, glutamine, tryptophane, glycine, valine, proline, sérine, tyrosine, arginine, histidine et des combinaisons de ceux-ci ;
un antioxydant ;
une vitamine ; ou
un minéral.

16. Produit laitier fermenté à longue durée de conservation préparé conformément au procédé selon l'une quelconque des revendications 1 à 15.

17. Produit laitier fermenté à longue durée de conservation selon la revendication 16, ayant une viscosité d'au moins 20 000 mPa.s (centipoise) en utilisant une broche numéro 6 Brookfield RV à 5 tr/min pendant 10 secondes.
